# EUROPEAN PATENT SPECIFICATION

(11) **EP 0 768 855 B1**
(45) Date of publication and mention of the grant of the patent: **17.11.1999**
(21) Application number: 95923472.5
(22) Date of filing: 03.07.1995
(51) Int. Cl.: A61F 13/44, A61L 15/54

(54) **DRESSING**
VERBAND
PANSEMENT

(30) Priority: 04.07.1994 GB 9413422; 22.09.1994 GB 9419077
(43) Date of publication of application: 23.04.1997
(73) Proprietor: Smith & Nephew plc, London WC2R 3BP (GB)
(72) Inventor: WARD, William, John, Hull HU5 3JP (GB); HUDSON, Simon, John, North Humberside HU14 3AY (GB)
(74) Representative: Gilholm, Stephen Philip
(86) International application number: GB9501551
(87) International publication number: WO9601096

(56) References cited:
- EP-A- 0 399 140
- EP-A- 0 424 132
- DE-A- 3 421 155
- GB-A- 716 923
- GB-A- 1 381 855
- GB-A- 2 169 906
- GB-A- 2 199 839
- US-A- 4 612 242

## Description

The present invention relates to radiopaque dressings, eg. for contaminant discrimination by x-ray detection equipment in manufacturing industries such as the food industry. The present invention is particularly applicable to industries employing automated detection processes operating at fast manufacturing process line speeds.

Dressings, and first aid dressings in particular, worn by personnel employed in food manufacturing industries are potential contaminants and there has therefore long been a need for such dressings to be detectable. Contaminant discrimination equipment can include ferrous and non ferrous metal detectors but generally such equipment suffers from the disadvantage that it is unsuitable for use in relation to metal packaging and at fast product and manufacturing process line speeds.

British Patent No.1381855 describes dressings which may be screened by a metal detector. Such dressings comprise a backing material of microporous polyvinyl chloride having a pressure-sensitive adhesive on one surface thereof, with an electromagnetically detectable particulate ferrous material incorporated in the backing material. In addition to being unsuitable for use in relation to metal packaging, such ferrous material dressings are also insufficiently sensitive to x-ray detectors. This document forms the preamble of independent claim 1.

X-ray detection is a further suitable means for identifying contaminant objects. X-ray detectable materials, in particular x-ray detectable surgical sponges, are known from British Patent Specification No.1575527 which describes elastomeric filaments loaded with from 40 to 90% w/w of powdered barium sulphate. The detection of such sponges is generally carried out by a manual inspection of an x-ray plate since barium sulphate has a relatively low attenuation. Therefore such filaments would need to be several millimetres thick to achieve the required degree of x-ray detectability at industrial line processing speeds. Thus, they would not be suitable for incorporation into thin x-ray detectable first aid dressings and may make the dressing uncomfortable to wear.

British Patent No.716923 describes surgical sponges and the like used for packing wound cavities comprising a sinuous monofilament of an x-ray detectable material.

British Patent Application No.2169906 describes radiopaque materials such as adhesive or coating materials comprising a radiopaque filter such as barium sulphate, a coupling agent and a polymeric material.

Thus known materials are not suitable for use in x-ray detectable dressings since the filaments would need to be relatively thick to provide the required high attenuation necessary for high speed x-ray detection.

We have now surprisingly found that tungsten, or salts thereof, may be used as a radiopaque material. In particular tungsten may be applied at a relatively low thickness whilst providing an appropriate attenuation. Tungsten is also advantageous since it is sufficiently non-toxic in contact with an open wound and it does not give toxic extracts in contact with wound exudates.

Thus according to the invention at least a portion of the dressing is radiopaque, the radiopacity being provided by tungsten or a salt thereof.

By the term dressing we mean, in particular, wound dressing, eg. first aid dressing, although it is intended that the present invention may also be useful in relation to tapes and closure strips also used in wound management. By the term radiopaque we mean impervious to electromagnetic radiation, eg. x-rays.

The dressing according to the invention is preferably an adhesive coated dressing, eg. a dressing comprising a backing layer, such as a film or fabric, and an adhesive layer, eg. a skin contacting adhesive layer. Film dressings are particularly preferred and especially thin film dressings.

Any conventionally known films may be used and such films may be microporous and/or moisture vapour permeable. Examples of such films include polyurethane films used in conventional wound dressings or polyvinyl chloride (PVC) films. However, other known films would also be suitable for use in the dressings of the invention.

The adhesive layer of the dressing may be formed from an adhesive which is conventionally used for contact with the skin. Suitable adhesives included polyvinyl alkyl ether adhesive and acrylate ester copolymer adhesives. Suitable adhesives are described in UK Patent No 1280631 and European Patent Nos.35399 and 51935. Preferably the adhesive is a polyvinyl ether adhesive or an acrylate ester copolymer adhesive formed by the copolymerisation of 2-ethylhexyl acrylate, butyl acrylate and acrylic acid. The adhesive layer of the dressing may be from 15 to 65µm thick, for example 20 to 40µm thick and is applied at a weight per unit area of 10 to 75gsm, more suitably 15 to 65gsm and preferably 25 to 40 gsm.

In a preferred dressing of the invention the dressing is provided with an absorbent pad on the surface of the adhesive layer. The absorbent pad may comprise any material conventionally used in first aid dressings, such as cotton wool, lint or foam.

In such wound dressings, and especially wound dressings which comprise a fibrous absorbent pad, the pad may also be provided with a net on the wound facing surface of the pad, to prevent contamination of the wound site by the pad material.

The whole of the dressing may be rendered radiopaque. Thus, the tungsten or a salt thereof may be present in the backing layer, in the adhesive layer or in an attached radiopaque strip. However, the inclusion of tungsten in the backing is generally expensive since relatively large amounts of tungsten or salts thereof may need to be used. Whilst it may be advantageous to include the tungsten in the adhesive layer, high loadings again may be expensive and will also hinder the adhesive qualities of the adhesive layer. Nevertheless, such dressings are to be considered within the scope of the present invention.

However, since tungsten and salts thereof are generally expensive, it is preferable that only a region of the dressing is rendered radiopaque. The size of the radiopaque region may vary depending on, inter alia, the nature of the tungsten used, achieving the desired attenuation, and the loading of tungsten in the dressing. Generally, the radiopaque region will comprise a small strip which may be from 0.1 to 1.0mm in width, preferably from 0.1 to 0.8mm, more preferably from 0.15 to 0.5mm and especially from 0.2 to 0.4mm. Since the strip width may vary it is preferable to consider a mean strip with width which may be from 0.1 to 0.5mm, preferably 0.2 to 0.4mm and especially 0.25 to 0.35mm, eg. 0.28mm.

Similarly, the depth of the strip may be from 0.1 to 1.0mm, preferably from 0.1 to 0.8mm, more preferably from 0.15 to 0.5mm and especially from 0.2 to 0.4mm.

In a particularly preferred embodiment of the invention the tungsten is loaded into a radiopaque strip which strip is located intermediate the adhesive layer and the absorbent pad of the dressing.

The tungsten or a salt thereof is preferably loaded into a carrier material. Conventionally known carrier materials may be used, but preferably the carrier material is an adhesive. Note however that when heavily loaded with tungsten an adhesive may lose much of it's adhesive properties and therefore reference to an adhesive carrier material is intended to refer to the properties of the carrier material prior to tungsten loading.

Thus the carrier material is preferably in the form of a thin strip and especially a thin strip of adhesive carrier material.

Tungsten loaded adhesive produces a thin x-ray detectable material which will retain some adherent properties. The adhesive acts as a binding agent for the tungsten, powder or a salt thereof.

In one embodiment of the invention the tungsten loaded adhesive carrier is spread onto a substrate. Substrates can be of any of, or any combination of paper, metal foil, polymeric film such as polyethylene or polypropylene. A preferred substrate is a metal and paper laminate, eg. an aluminium and paper laminate. This provides the necessary support to allow the dressing to be processed. Preferably, although not essentially, the tungsten loaded adhesive carrier is applied to the paper side of the laminate and the aluminium side is adhered onto the adhesive layer of the dressing. When a laminate is used the dimensions of the radiopaque strip hereinbefore described shall refer only to the tungsten loaded carrier and not the laminate. The loaded carrier may be applied in any pattern to any of the substrates or components of a dressing.

The adhesive carrier material may be the same as that used in the adhesive layer of the dressing. However, a relatively high viscosity adhesive is preferred, thus allowing the tungsten to remain dispersed within the carrier. More particularly, the adhesive carrier is a rubber based adhesive.

The adhesive carrier may be loaded with tungsten metal powder, tungsten trioxide or any other suitable tungsten salt. The tungsten or a salt thereof may be in any suitable form but preferably is in powder form. Tungsten metal powder is especially preferred.

The tungsten powder may have a mean particle size of from 4.5 to 5.5µm, eg. 5µm and a Scott density of from 30 to 70 g/inch³, preferably 40 to 70 g/inch³.

Physical strength and conformability provide advantages for manufacturing and processing.

The percentage of tungsten in the adhesive carrier is inversely proportional to the thickness of the tungsten loaded carrier in a dressing which is required for x-ray detection.

Suitably the amount of tungsten which can be loaded into a carrier, eg. an adhesive, to be detectable when incorporated in a first aid dressing is from 10% to 90% w/w, preferably from 30 to 90% w/w, more preferably from 50 to 90% w/w and especially from 70 to 90% w/w. The above values are calculated for the use of tungsten metal powder and based on the dried carrier material.

For example, 1 part by weight of a suitable wet adhesive can be mixed with 2 parts by weight of tungsten metal powder to form the tungsten loaded adhesive detectable element compound.

A strip of 0.10mm to 1mm thickness of tungsten loaded adhesive is suitably applied in a first aid dressing. The use of this small amount of compound allows the dressing to be a low cost disposable device.

Up to 90% by weight of tungsten may be loaded into a rubber resin adhesive. Such adhesives are well known and are composed of natural rubber, natural resin and fillers, antioxidants and softeners in a suitable solvent such as petrol. When the constituents are mixed the mixture may be spread and surplus solvent dried off.

It is preferable that a dressing according to the present invention should also be metal detectable. However, tungsten alone is not sufficiently detectable by metal detectors used in industries. Iron can therefore be incorporated into the dressing to ensure that a dressing can also be detectable by ferrous detectors.

Thus, according to a further feature of the invention we provide a dressing as hereinbefore described which is sensitive to a metal detector.

The iron may be incorporated in a similar manner to the tungsten, eg. in a strip intermediate the adhesive layer and the absorbent pad. However, since iron is less expensive than tungsten the size of a metal detectable strip need not be as small as that of a tungsten strip. However, it is preferred that the iron is dispersed in the backing film, eg. a PVC film. Thus, particularly preferred films are those described in British Patent No. 1381855 which is incorporated herein by reference.

The present invention thus serves to provide an x-ray detectable first aid dressing which uses an economic quantity of tungsten in a highly conformable form that is safe, easy to produce and comfortable to wear.

Although radiopaque adhesives are known, it is novel to use tungsten or a salt thereof in such adhesives.

Thus according to a further feature of the invention we provide a radiopaque adhesive characterised in that the radiopacity is provided by tungsten or a salt thereof.

Similarly a tungsten loaded adhesive spread on a carrier, eg. a laminate, as hereinbefore described are also novel.

According to the invention we also provide a process for the manufacture of a dressing as hereinbefore described, which comprises;
(i) spreading a radiopaque adhesive carrier onto a substrate;
(ii) adhering a strip of the substrate to an adhesive layer of a dressing, or laminating the substrate to an absorbent pad, and
(iii) adhering an absorbent pad or the substrate/absorbent pad laminate to the adhesive layer of a dressing such that the absorbent pad covers the substrate.

The present invention will now be further described with reference to the non-limiting accompanying drawings in which
Figure 1 is a top view of a first aid dressing according to the present invention with net and pad omitted,
Figure 2 is a longitudinal section through a dressing as shown in Figure 1 with net and pad shown, and
Figure 3 is an x-ray photograph of a dressing of the invention.

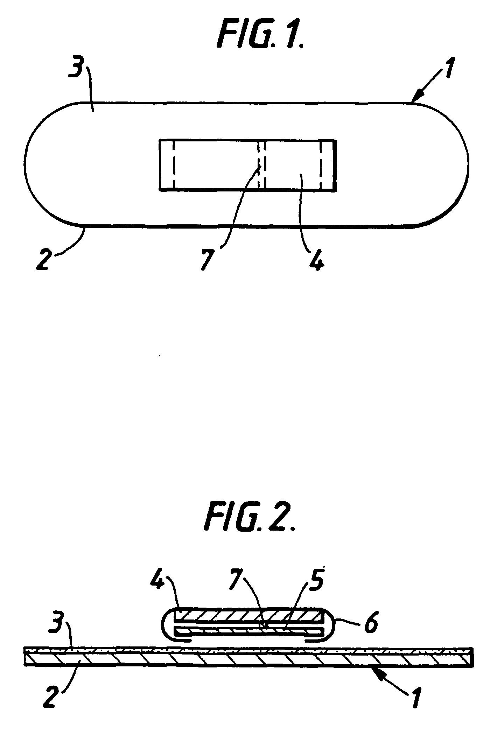

As can be seen from Figures 1 and 2, a first aid dressing (1) consists of a backing layer (2) having an adhesive layer (3) on one surface thereof with an absorbent pad (4) secured on the surface of the adhesive layer (3). To make the dressing detectable by metal detectors, the substrate can incorporate particulate ferrous material (not shown) and/or an aluminium foil/paper laminate (5) can be incorporated intermediate the adhesive layer (3) and the pad (4). The pad (4) and laminate (5) can be enclosed by a net (6). To make the dressing x-ray detectable by detection equipment used in manufacturing industries, a strip of tungsten loaded adhesive (7) is incorporated on the surface of the aluminium foil/paper laminate (5) intermediate the pad (4) and the laminate (5).

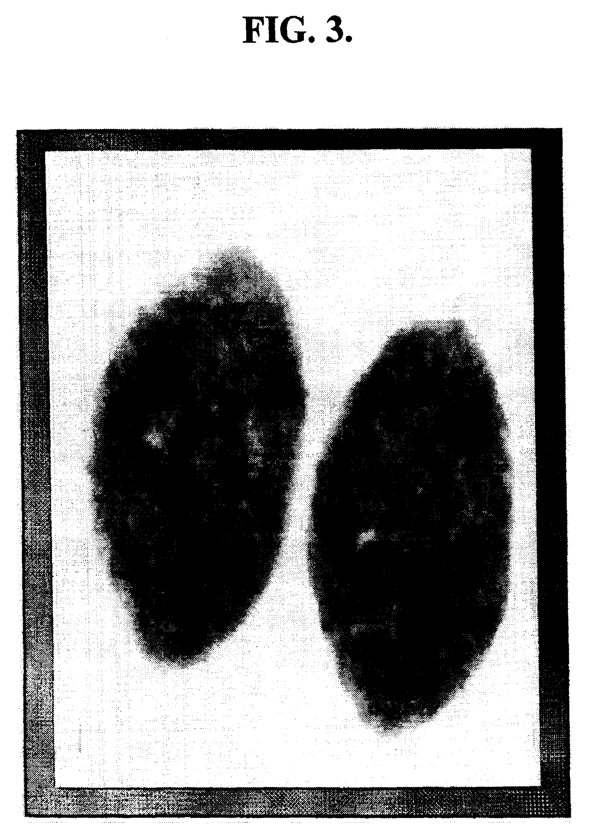

With reference to Figure 3, an x-ray of a conventional first aid dressing (8) is illustrated and in a dressing (1) the tungsten loaded adhesive (7) can be seen.

The invention will now be described by way of example only in Example 1.

### Example 1

Strips of an adhesive carrier, loaded with 90% w/w tungsten were coated onto aluminium/paper laminate and non-woven pad material, which is thick and sort enough to mask the strip was laminated to the adhesive.

To prepare tungsten loaded adhesive, the tungsten is added to the wet mixture before spreading and drying. A 1:2 ratio weight of wet rubber adhesive to tungsten powder gave a suitable adhesive that mixed well, did not settle too fast, flowed and had enough adhesion when dry to laminate pad to foil.

The aluminium/paper laminate was cut with the pad to which it is lightly attached by the strip of tungsten loaded adhesive. A layer of net was wrapped round the pad and x-ray and metal detectable element and the detectable element side secured to the adhesive side of the main dressing thereby binding the pad securely to the main dressing, the net also serving its main design function of reducing adhesion of the pad to the wound. The metal/x-ray detectable element is thus attached securely to the dressing and is not removable by normal wear and tear.

Strips of tungsten loaded adhesive of 10mm x 1mm x 0.3mm thickness were incorporated into x-ray detectable first aid dressings.

X-ray detectable first aid dressings (FAD) were detectable by GK Graseby Intertest CDX equipment. Detector settings were 40KV, 4.0mA and scan speed was 1m/min. These conditions reflect those commonly used in manufacturing industries.

Tungsten oxide combination dispersed in rubber was acceptably detectable at thicknesses above 3 thou (0.12mm), with 6 thou (0.24mm) +/- 1 thou being the preferred range. This is the reference sample as referred to in Table 1 and this preferred range was confirmed by direct measurement on the industrial x-ray detection equipment as described above. Table 1 gives results for each strip spread across the samples tested, in terms of thickness and the x-ray beam power reduction settings that gave the start (first detection) and end (no further increase in detection) for the strips. The lower the numerical value of the start and end results, the higher the detectability of the dressing element.

Detectable strip thickness of 0.24mm of tungsten loaded adhesive behind pad material in a dressing as described above appears to be not noticeable to the wearer and the detectability of the dressing by standard ferrous detector methods was not compromised.

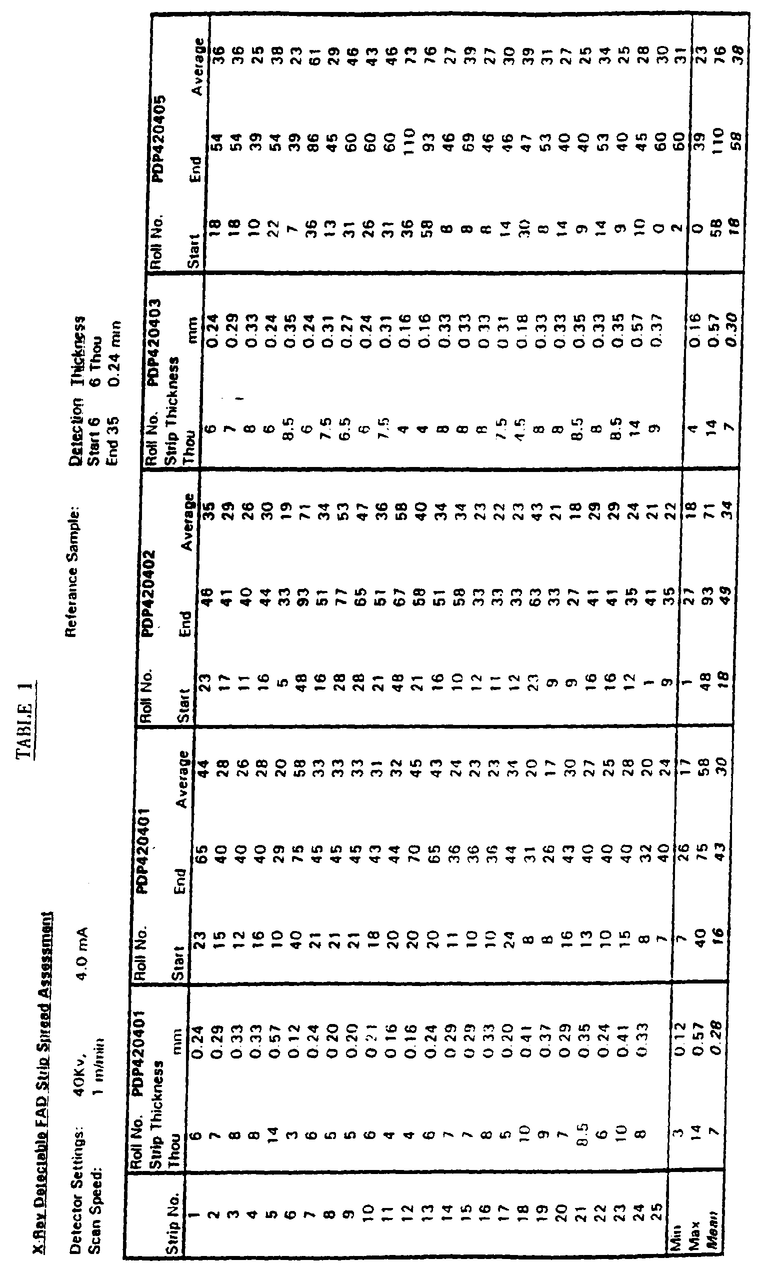

## Claims

1. A dressing (1) comprising a backing layer (2) and an adhesive layer (3), characterised in that at least a portion of said dressing is radiopaque and in that the radiopacity is provided by tungsten or a salt thereof.

2. A dressing according to claim 1 wherein the dressing (1) is provided with an absorbent pad (4) on the surface of the adhesive layer (3).

3. A dressing according to claim 1 wherein the radiopaque portion comprises tungsten or a salt thereof in a carrier material.

4. A dressing according to claim 3 wherein the carrier material is an adhesive carrier strip (7).

5. A dressing according to claims 3 or 4 wherein the carrier material is located intermediate the adhesive layer (3) and the absorbent pad (4).

6. A dressing according to claim 4 wherein the carrier strip (7) is on a substrate.

7. A dressing according to claim 3 wherein the carrier material is from 0.1 to 1.0mm thick.

8. A dressing according to claim 3 wherein the carrier material is from 0.1 to 1.0mm wide.

9. A dressing according to claim 3 wherein the amount of tungsten loaded into the carrier is from 10% to 90% w/w.

10. A dressing according to claim 1 wherein the tungsten or a salt thereof is in the form of powdered metal tungsten.

11. A dressing according to claim 1 wherein at least a portion of the backing layer (2) contains a detectable metal.

12. A dressing according to claim 11 wherein the detectable metal is iron.

13. A process for the manufacture of a dressing according to any one of the preceding claims in which radiopacity is provided by tungsten or a salt thereof which comprises;
(i) spreading a radiopaque adhesive onto a substrate,
(ii) adhering a strip of the substrate to an adhesive layer of a dressing, or laminating the substrate to an absorbent pad, and
(iii) adhering an absorbent pad or the substrate absorbent pad laminate to the adhesive layer of a dressing such that the absorbent pad covers the substrate.

## Patentansprüche

1. Verband (1) umfassend eine Rückschicht (2) und eine Haftschicht (3), dadurch gekennzeichnet, daß wenigstens ein Teil des Verbandes strahlenundurchlässig ist und daß die Strahlenundurchlässigkeit durch Wolfram oder ein Salz davon geliefert wird.

2. Verband gemäß Anspruch 1, wobei der Verband (1) auf der Oberfläche der Haftschicht (3) mit einem absorbierenden Kissen (4) ausgestattet ist.

3. Verband gemäß Anspruch 1, wobei der strahlenundurchlässige Teil Wolfram oder ein Salz davon in einem Trägermaterial umfaßt.

4. Verband gemäß Anspruch 3, wobei das Trägermaterial ein Klebstoffträgerstreifen (7) ist.

5. Verband gemäß Anspruch 3 oder 4, wobei sich das Trägermaterial zwischen der Haftschicht (3) und dem absorbierenden Kissen (4) befindet.

6. Verband gemäß Anspruch 4, wobei sich der Trägerstreifen (7) auf einem Substrat befindet.

7. Verband gemäß Anspruch 3, wobei das Trägermaterial 0,1 bis 1,0 mm dick ist.

8. Verband gemäß Anspruch 3, wobei das Trägermaterial 0,1 bis 1,0 mm breit ist.

9. Verband gemäß Anspruch 3, wobei die Menge des dem Träger zugesetzten Wolframs 10 bis 90 Gew.-% ist.

10. Verband gemäß Anspruch 1, wobei das Wolfram oder ein Salz davon in Form von Wolframmetallpulver vorliegt.

11. Verband gemäß Anspruch 1, wobei wenigstens ein Teil der Rückschicht (2) ein nachweisbares Metall enthält.

12. Verband gemäß Anspruch 11, wobei das nachweisbare Metall Eisen ist.

13. Verfahren zur Herstellung eines Verbandes gemäß einem der vorangehenden Ansprüche, wobei die Strahlenundurchlässigkeit durch Wolfram oder ein Salz davon geliefert wird, umfassend das
(i) Ausstreichen eines strahlenundurchlässigen Klebstoffs auf ein Substrat,
(ii) Kleben eines Streifens aus dem Substrat an eine Haftschicht eines Verbandes oder Laminieren des Substrates an ein absorbierendes Kissen und
(iii) Kleben eines absorbierenden Kissens oder des Laminates aus dem Substrat und absorbierenden Kissen an die Haftschicht eines Verbandes, so daß das absorbierende Kissen das Substrat bedeckt.

## Revendications

1. Pansement (1) comprenant une couche de renforcement (2) et une couche adhésive (3), caractérisé en ce qu'au moins une partie dudit pansement est radio-opaque et en ce que la radio-opacité est fournie par du tungstène ou un sel de celui-ci.

2. Pansement suivant la revendication 1, dans lequel le pansement (1) est pourvu d'un tampon absorbant (4) sur la surface de la couche adhésive (3).

3. Pansement suivant la revendication 1, dans lequel la partie radio-opaque comprend du tungstène ou un sel de celui-ci dans un matériau de support.

4. Pansement suivant la revendication 3, dans lequel le matériau de support est une bande de support adhésive (7).

5. Pansement suivant les revendications 3 ou 4, dans lequel le matériau de support est situé entre la couche adhésive (3) et le tampon absorbant (4).

6. Pansement suivant la revendication 4, dans lequel la bande de support (7) est sur un substrat.

7. Pansement suivant la revendication 3, dans lequel le matériau de support a une épaisseur de 0,1 à 1,0 mm.

8. Pansement suivant la revendication 3, dans lequel le matériau de support a une largeur de 0,1 à 1,0 mm.

9. Pansement suivant la revendication 3, dans lequel la quantité de tungstène chargée dans le support est de 10% à 90% en poids/poids.

10. Pansement suivant la revendication 1, dans lequel le tungstène ou un sel de celui-ci est sous la forme de tungstène métallique pulvérulent.

11. Pansement suivant la revendication 1, dans lequel au moins une partie de la couche de renforcement (2) contient un métal détectable.

12. Pansement suivant la revendication 11, dans lequel le métal détectable est le fer.

13. Procédé pour la fabrication d'un pansement suivant l'une quelconque des revendications précédentes dans lequel une radio-opacité est fournie par du tungstène ou un sel de celui-ci, qui comprend :
(i) l'étalement d'un adhésif radio-opaque sur un substrat,
(ii) le collage d'une bande du substrat sur une couche adhésive d'un pansement, ou le feuilletage du substrat sur un tampon absorbant, et
(iii) le collage d'un tampon absorbant ou du feuilleté de substrat et de tampon absorbant sur la couche adhésive d'un pansement de telle sorte que le tampon absorbant recouvre le substrat.
